# FASCICULE DE BREVET EUROPEEN

(11) **EP 2 027 246 B1**
(45) Date de publication et mention de la délivrance du brevet: **21.07.2010**
(21) Numéro de dépôt: 07729278.7
(22) Date de dépôt: 18.05.2007
(51) Int. Cl.: C12M 1/00

(54) **DISPOSITIF DE CULTURE DE CELLULES OU MICRO-ORGANISMES**
VORRICHTUNG ZUR KULTIVIERUNG VON ZELLEN ODER MIKROORGANISMEN
DEVICE FOR CULTURE OF CELLS OR MICRO-ORGANISMS

(30) Priorité: 19.05.2006 FR 0604525
(43) Date de publication de la demande: 25.02.2009
(73) Titulaire: Centre National de la Recherche Scientifique- CNRS, 75794 Paris (FR)
(72) Inventeur: OUAZZANI, Jamal, F-91300 Massy (FR); CORTIAL, Sylvie, F-91940 Les Ulis (FR); SERGENT, Didier, F-91220 Bretigny S/Orge (FR); LOPES, Philippe, F-91530 St Maurice Montcouronne (FR)
(74) Mandataire: Texier, Christian
(86) Numéro de dépôt international: PCT/EP2007/054834
(87) Numéro de publication internationale: WO 2007/135098

(56) Documents cités:
- EP-B1- 1 131 404
- WO-A-94/04273
- BE-A- 737 189
- DE-A1- 4 028 871
- DE-C1- 4 443 902
- GB-A- 1 360 968
- JP-A- 11 089 556
- US-A- 3 562 114
- US-A- 4 212 949

## Description

La présente invention concerne le domaine de la culture de cellules. Elle s'applique en particulier à la culture de micro-organismes. Cependant, elle n'est pas limitée à cette application particulière et s'étend à la culture de tout type cellulaire (cellules végétales, cellules d'insectes voir même des cellules animales).

L'homme de l'art sait en particulier que la recherche et le développement de molécules d'intérêts, à partir de substances naturelles, constituent un enjeu majeur dans le domaine thérapeutique.

Cette recherche nécessite des équipements permettant la culture de micro-organismes.

De nombreux équipements à cet effet ont déjà été proposés.

Un exemple d'équipement connu est décrit dans le document EP-A-1131404. Cet équipement a des limites. En particulier il ne permet pas de culture en milieu liquide ce qui exclut la culture de cellules d'insectes ou de cellules animales.

Cependant, aucun des équipements connus ne donne totalement satisfaction à ce jour, notamment en s'adaptant à différents types cellulaires, en permettant des cultures sur des supports de type gélose nutritive et en cumulant les différentes étapes d'une culture cellulaire.

Dans ce contexte, la présente invention a pour objectif de proposer un nouvel équipement permettant d'améliorer la situation.

Cet objectif est atteint selon la présente invention grâce à un dispositif destiné à la culture de cellules ou micro-organismes, caractérisé par le fait qu'il comprend une enceinte constituée d'une cuve et d'un couvercle, adaptée pour définir à l'état fermé un volume étanche sous pression contrôlée et un ensemble conçu pour être placé à l'intérieur de l'enceinte tout en étant susceptible d'être retiré de celle-ci lors de l'ouverture du couvercle, ledit ensemble comprenant un châssis support et une pluralité de plateaux portés par le châssis support, l'enceinte comprenant par ailleurs des moyens conçus pour assurer successivement, la stérilisation de son contenu, l'ensemencement d'un milieu de culture placé dans les plateaux et un contrôle de l'atmosphère de l'enceinte propre à permettre la culture de cellules ou micro-organismes.

Comme on l'exposera par la suite plus en détail, la structure ainsi proposée selon la présente invention, permet d'opérer toutes les étapes essentielles pour la culture des micro-organismes au sein même de l'enceinte. Ainsi, la présente invention permet d'éviter toute contamination avant et en au cours de la culture.

Selon une autre caractéristique avantageuse de l'invention, la présente invention comprend également des moyens conçus pour assurer un traitement après la culture, de préférence un traitement thermique apte à sécher le milieu de culture ainsi que les micro-organismes cultivés.

Selon une autre caractéristique avantageuse de la présente invention, l'interface définie entre la cuve et le couvercle de l'enceinte est une interface comprenant un joint annulaire d'étanchéité.

Selon une autre caractéristique avantageuse de la présente invention, le châssis support est relié au couvercle de l'enceinte.

Selon une autre caractéristique avantageuse de la présente invention, l'enceinte est conçue pour contrôler un flux de gaz injecté dans la cuve, notamment un flux de vapeur, d'air, d'oxygène ou de tout autre gaz qui assure de manière optimale la culture, sans risque d'entraînement de micro-organismes.

D'autres caractéristiques, buts et avantages de la présente invention apparaîtront à la lecture de la description détaillée qui va suivre et en regard des dessins annexés donnés à titre d'exemples non limitatifs et sur lesquels :
- la figure 1 représente une vue schématique externe d'un dispositif conforme à la présente invention,
- la figure 2 illustre l'extraction de l'ensemble constitué d'un châssis support et de plateaux hors de la cuve lors de l'ouverture du couvercle,
- les figures 3, 4 et 5 illustrent trois étapes de démontage dudit ensemble afin d'accéder aux plateaux,
- la figure 6 représente une vue latérale d'une palle utilisée pour homogénéiser le milieu porté par un plateau, ou pour étaler une préculture sur celui-ci.
- la figure 7 représente schématiquement des moyens prévus sur la cuve pour assurer l'ensemencement des plateaux,
- la figure 8 représente une vue schématique, partiellement en coupe verticale, du dispositif conforme à la présente invention, et
- la figure 9 représente une vue schématique en coupe horizontale du même dispositif.

Comme on l'a évoqué précédemment, le dispositif conforme à la présente invention se compose essentiellement d'une enceinte 100 et d'un ensemble extractible 200.

L'enceinte 100 est composé d'une cuve 110 et d'un couvercle 150.

L'ensemble 200 est composé d'un châssis support 210 et de plateaux 250.

L'enceinte 100 présente une symétrie générale de révolution autour d'un axe vertical O-O.

La cuve 110 est constituée d'une paroi 112 cylindrique de révolution autour de l'axe O-O, fermée à sa base par une calotte 114 concave vers l'extérieur, typiquement sensiblement hémisphérique.

De préférence, sur la quasi totalité de sa hauteur, la paroi cylindrique 112 est recouverte sur l'extérieur d'une chambre annulaire 113 formant double enveloppe. Une telle chambre permet lorsqu'elle est alimentée en un fluide à température contrôlée, de contrôler de manière homogène la température du volume interne de l'enceinte.

Ce contrôle en température peut être utilisé, bien entendu en adaptant et en faisant évoluer la température du fluide alimentant la chambre 113, l'ensemble des étapes mises en oeuvre, à savoir stérilisation, ensemencement, culture, voir étape ultérieure, notamment de séchage.

Plus précisément, comme on le voit sur les figures annexées, de préférence la chambre annulaire 113 ne couvre pas la totalité de la périphérie de la cuve 112. La chambre annulaire 113 possède une section droite horizontale en C qui couvre environ 310 à 320° autour de la paroi 112.

Au niveau de l'espace libre défini entre les deux extrémités périphériques de la chambre annulaire 113, c'est-à-dire l'espace où la paroi 112 est directement accessible, cette dernière est munie de préférence d'un hublot 120 et de moyens 130 conçus pour assurer l'ensemencement d'un milieu de culture portés par les plateaux 250. Le hublot 120 présente ainsi de préférence une forme oblongue dont la grande dimension s'étend verticalement sur toute la hauteur de la paroi 112 située en regard des plateaux 250.

Les moyens 130 peuvent être l'objet de différentes variantes. Ils sont formés de préférence de bouchons 132 en matériau élastomère ou équivalents susceptibles d'être traversés par des aiguilles adaptées, elles-mêmes reliées à l'extérieur de l'enceinte à une source d'ensemencement, pour assurer un ensemencement contrôlé en micro-organismes d'un milieu de culture porté par les plateaux 250. Pour cela, l'extrémité desdites aiguilles situées à l'intérieur de l'enceinte doivent être situées au-dessus des plateaux 250, et ce de préférence sur une zone contrôlée avec précision. Ces aiguilles ou des aiguilles de longueur différente permettent aussi l'addition de toute solution dans les plateaux ainsi que le prélèvement stérile par aspiration de tout ou partie du contenu du plateau.

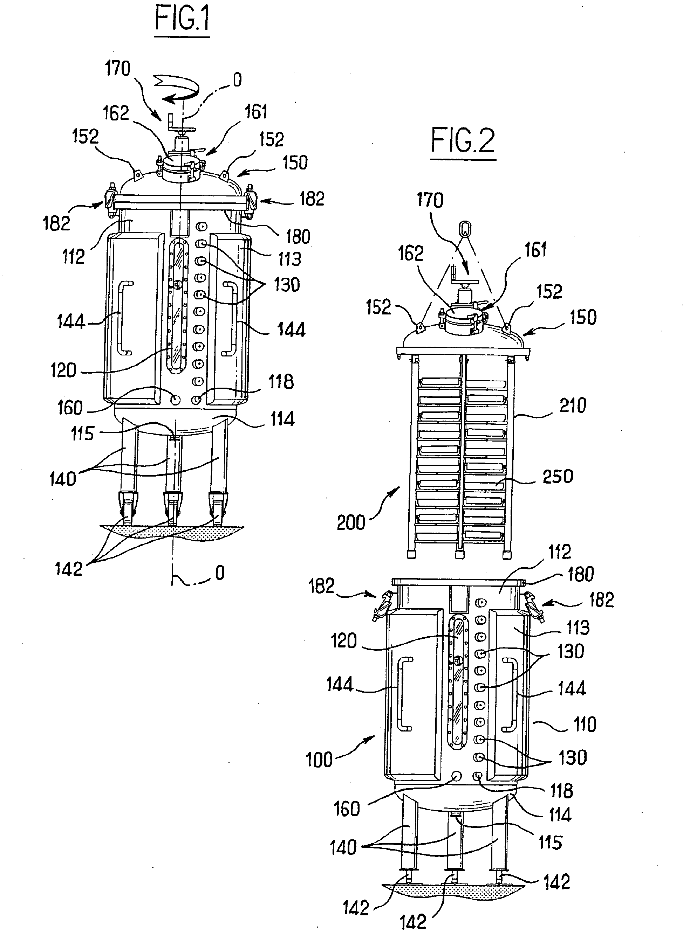

Un tel contrôle peut être opéré d'une part, visuellement grâce au hublot 120. En effet, comme on le voit sur les figures 1 et 2, le hublot 120 est adjacent des moyens 130. D'autre part, ce contrôle peut être opéré en veillant à contrôler avec précision la longueur des aiguilles introduites dans l'enceinte, ceci par exemple en contrôlant la position d'un repère prévu sur les aiguilles par rapport au bouchon 130.

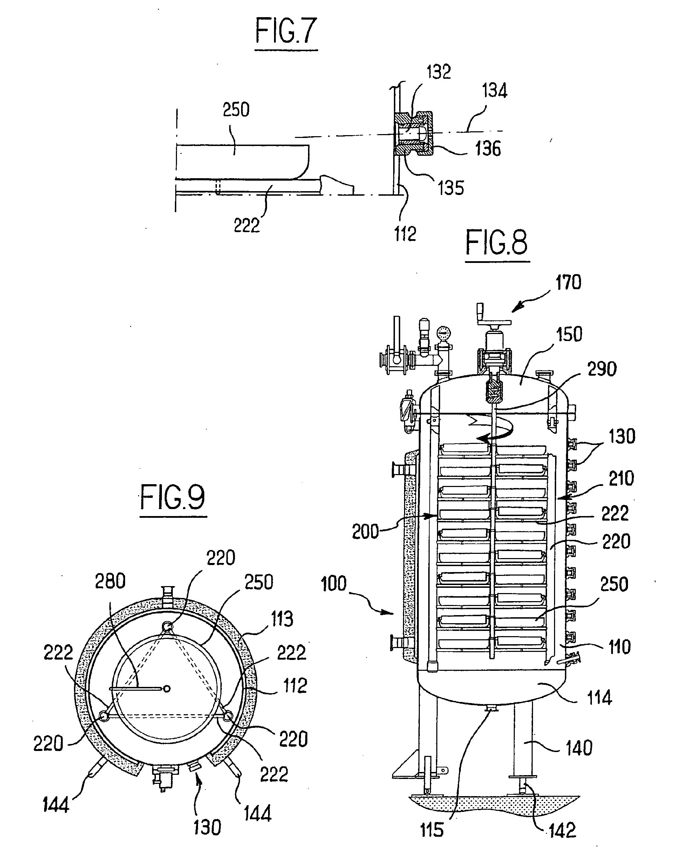

Comme on l'aperçoit sur la figure 7, de préférence chaque moyen 130 comprend un bloc élastomère 132 généralement cylindrique de révolution autour d'un axe 134 globalement radial par rapport à l'axe O-O mais légèrement incliné sur l'horizontal, vers le bas en rapprochement de l'axe O-O. Les blocs élastomère 132 sont avantageusement placés dans une cage 135 associée à une bague 136 amovible permettant le remplacement des blocs 132 après plusieurs utilisations.

L'homme de l'art comprendra que de tels bouchons 130, en matériau élastomère, assurent automatiquement l'étanchéité de l'enceinte lors du retrait de l'aiguille.

Il est prévu un nombre de moyens 130, répartis sur la hauteur de la cuve 110, égale au nombre de plateaux 250 portés par le châssis support 210.

Bien entendu en variante, on pourrait prévoir une chambre annulaire 113 couvrant la totalité de la périphérie de la paroi 112 et de placer par conséquent le hublot 120 et les moyens 130 sur cette chambre 113 en veillant à assurer l'étanchéité à ce niveau.

La calotte inférieure 114 est montée de préférence sur un ensemble de pieds 140, typiquement trois pieds 140, pourvus chacun d'une roue 142 à leur base. Cette disposition permet de déplacer aisément le dispositif, par exemple entre un lieu de chargement et déchargement de l'ensemble 200 (comme schématisé sur la figure 2), nécessitant une grande hauteur, et un lieu de culture ou de stockage, à l'état fermé (comme schématisé sur la figure 1), acceptant une hauteur d'installation inférieure.

Pour faciliter ce déplacement, la cuve 110 est de préférence munie de poignées 144, situées par exemple sur l'extérieur de la chambre annulaire 113.

De préférence, la calotte 114 comporte à sa base un orifice de vidange 115 obturé par tous moyens appropriés susceptibles d'ouverture à la demande.

Selon le type de culture et le processus de culture retenu, la calotte 114 peut recevoir dans son volume interne un fluide participant au contrôle de l'atmosphère interne de l'enceinte.

L'orifice 115 permet de vidanger aisément un tel fluide, voir de prélever pour analyses éventuelles, un échantillon de ce fluide.

La cuve 112 est munie de plus d'au moins une entrée 160 permettant d'injecter un fluide ou gaz approprié (oxygène, air comprimé, vapeur d'eau ...) que ce soit pendant les étapes de stérilisation, ensemencement, culture, ou toute autre étape notamment au cours d'un éventuel traitement ultérieur par séchage. L'entrée 160 est de préférence prévue en partie inférieure de la paroi 112, à proximité de la calotte 114.

On a représenté sur les figures annexées, un dispositif comportant également une sonde de température 118, adaptée pour mesurer la température interne de l'enceinte, portée par la paroi 112 à proximité de la calotte 114.

Une telle sonde 118 peut être remplacée par tous moyens appropriés, par exemple et non limitativement, des moyens de mesure de température adaptés pour être placés au sein même du milieu de culture portés par les plateaux 250. Dans ce cas, de préférence, ces moyens de température sont du type à transmission sans fil.

Comme on le voit sur les figures annexées, la cuve 110 est munie à sa partie supérieure d'une collerette 180 servant d'appui au couvercle 150 et d'une série de moyens 182 adaptés pour assurer le verrouillage du couvercle 150 avec étanchéité est tenu en pression. De tels moyens 182 classiques en soit ne seront pas décrits dans le détail par la suite.

Le couvercle 150 est formé essentiellement d'une calotte convexe vers l'extérieur, symétrique de la calotte 114, c'est-à-dire sensiblement hémisphérique.

Un joint d'étanchéité, non représenté dans le détail sur les figures annexées est susceptible de faire l'objet de tout mode de réalisation approprié, est intercalé entre la collerette 180 de la cuve 112 et la périphérie de la base du couvercle 150. L'homme de l'art notera que la présente invention définit ainsi une interface annulaire de configuration simple permettant de garantir à la fois une parfaite étanchéité et une parfaite intégrité ou stérilisation, ce que ne permettrait pas des moyens d'ouverture d'une enceinte du type porte pivotante nécessitant des charnières ou équivalents dont l'étanchéité et la stérilisation sont complexes à réaliser.

Le couvercle 150 est muni de plusieurs points d'accrochage 152 permettant de soulever le couvercle, et le cas échéant, l'ensemble 200, par un palan ou équivalent.

Comme on le voit sur les figures, le couvercle 250 est muni d'un moyen d'accès type trou d'homme ou équivalents 161 comportant une trappe 162 susceptible d'ouverture à la demande et de fermeture étanche.

Le couvercle 150 peut être muni d'autres accessoires. On n'a en particulier illustré sur les figures annexées, un moyen 170 centré sur l'axe O-O et adapté pour assurer sur demande l'entraînement à rotation d'une tige 290 permettant d'entraîner à rotation des palles 280.

De tels moyens 170 peuvent être formés de moyens d'entraînement manuel à manivelle comme illustré sur les figures annexées ou de moyens d'entraînement motorisé associés à une programmation permettant de contrôler vitesse, durée et séquences de rotation.

Le couvercle 150 peut être muni de moyens d'éclairage permettant d'éclairer sélectivement le volume interne de l'enceinte, par exemple pendant les séquences de chargement, ensemencement, voir si nécessaire tout au long de la culture des micro-organismes. Il peut comporter également des moyens d'observation de l'intérieur de la cuve. Dans le cas où la cuve 110 est réalisée en acier inoxydable, sa surface interne forme réflecteur permettant une bonne homogénéité d'éclairage de l'ensemble du volume interne de l'enceinte à partir d'une source limitée portée par le couvercle. Le cas échéant, un système d'éclairage peut également être prévu au niveau du hublot pour faciliter l'observation du volume interne de l'enceinte. Un tel moyen d'éclairage peut être formé d'une rampe s'étendant sur toute la hauteur du hublot 120 ou d'une source de sections limitées montée à translation, de préférence sur l'extérieur de la cuve 110, le long du hublot 120, pour éclairer sélectivement l'un des plateaux 250.

Le châssis support 210 peut faire l'objet de nombreuses variantes. Il est composé de préférence de plusieurs colonnes verticales 220, typiquement trois colonnes 220 équi-réparties angulairement, reliées entre elles par des jeux de traverses ou barreaux horizontaux 222 servant de support aux plateaux 250.

Selon le mode de réalisation non limitatif illustré sur les figures annexées, les barreaux 222 définissent dix étages superposés adaptés pour recevoir chacun un plateau réceptif 250.

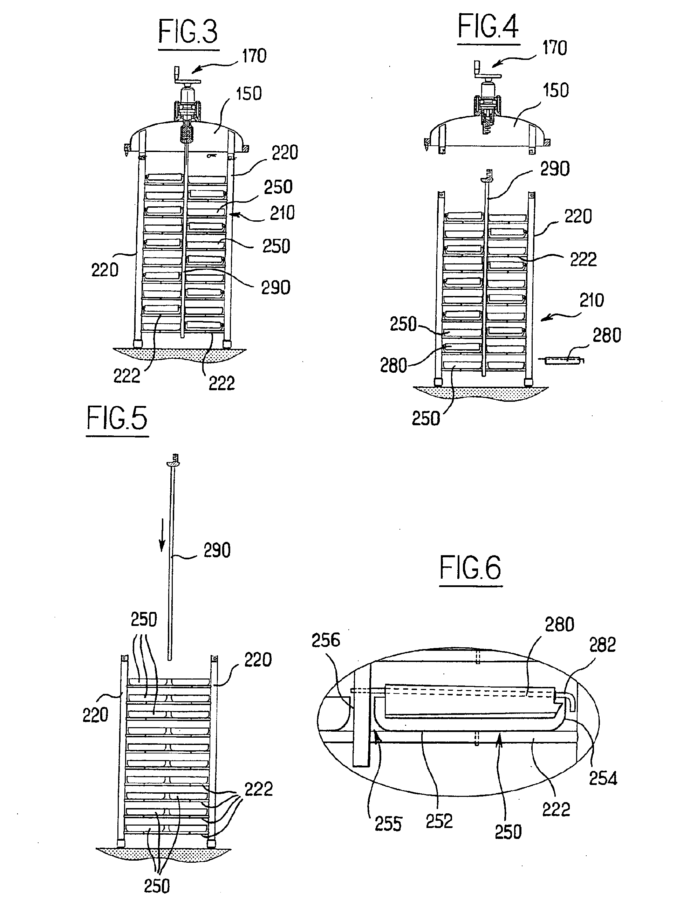

L'extraction du châssis 210 et des plateaux 250 portés par celui-ci peut être réalisé par tous moyens appropriés, par exemple en reliant les extrémités supérieures des colonnes 220 à un palan, après retrait du couvercle 150. Cependant, de préférence, comme illustré sur les figures 3 à 5, il est prévu des moyens de liaison entre les extrémités supérieures des colonnes 220 et le couvercle 150, par exemple à base de goupilles ou équivalents. Ainsi, l'extraction du châssis support 210 et des plateaux 250 est assurée lors de l'élévation du couvercle 150 comme on le voit sur la figure 2.

On notera qu'avantageusement, les moyens de liaison entre les extrémités supérieures des colonnes 220 et le couvercle 150 sont des moyens amovibles permettant de séparer châssis support 220 et couvercle 150 comme on le voit sur la figure 4.

Chaque plateau 250 est de préférence globalement cylindrique de révolution. Il comprend une base plane circulaire 252 surmontée à sa périphérie d'une jupe cylindrique 254. La hauteur des jupes 254 peut être variable. Elle est typiquement d'une hauteur de l'ordre de 5cm pour un diamètre de plateau de l'ordre de 50cm. Une telle structure de plateau est adaptée pour recevoir tout type de milieux de cultures, solides, semi-solides ou liquides, bien que la présente invention soit dédiée préférentiellement à une culture sur milieu de type gélose nutritive.

L'homme de l'art appréciera que les dimensions et le nombre de plateaux précédemment évoqués, bien entendu non limitatifs, permettent une culture simultanée sur une surface de l'ordre de 2m², bien supérieure aux surfaces autorisées par les dispositifs les plus classiques disponibles de nos jours, notamment sous forme de boîtes de Pétri.

Le dispositif conforme à la présente invention est également équipé de moyens conçus pour assurer l'homogénéisation du milieu de culture et/ou de l'ensemencement de celui-ci. Ces moyens peuvent faire l'objet de nombreuses variantes. Ils sont de préférence formés de palles ou râteaux 280 portés respectivement par des tiges secondaires 282 s'étendant radialement par rapport à la tige verticale principale 290 précédemment décrites.

Bien entendu, le dispositif contient au moins une tige 282 et au moins une palle 280 associée, répartie sur la longueur de la tige 290, pour chacun des plateaux 250. Comme on le voit sur la figure 6, l'extrémité libre radialement externe des tiges 282 peut reposer sur le bord supérieur des jupes 254 des plateaux 250 pour assurer un positionnement précis des palles 280 par rapport aux plateaux 250.

Les palles 280 peuvent être fixées en position sur les tiges 282. Elles sont cependant montées de préférence librement pivotantes sur ces tiges 282, autour de l'axe longitudinal de ces dernières, afin de balayer la surface du milieu de culture, sans pénétrer celui-ci.

Le cas échéant, les palles 280 précédemment décrites adaptées pour étaler le matériau d'ensemencement à la surface du milieu de culture peut être remplacé par des moyens de brassage. Dans ce cas, des moyens remplaçant les palles 280 sont liées rigidement au tige support 182 sans possibilité de rotation par rapport à l'axe longitudinal de ces dernières.

Comme on le voit sur les figures annexées, la présence des moyens 290, 282, 280 exige que la tige principale 290 traverse les plateaux 250. Pour cela, les plateaux 250 possèdent également en leur centre un orifice 255 surmonté d'une jupe cylindrique 256 de même hauteur que la jupe périphérique externe 254.

Pour retirer les plateaux 250, on procède essentiellement comme suit, une fois le couvercle 150 et le châssis support 210 retirés. Le couvercle 150 est séparé du châssis support 210 (figure 4). De préférence, les palles 280 et tiges supports 282 sont séparées de la tige principale 290 (figure 4). Enfin, la tige 290 est retirée par le haut (figure 5). Les plateaux 250 peuvent alors être retirés par translation horizontale.

En variante, on peut prévoir que chaque plateau 250 possède une gorge radiale dans sa base 252 reliant l'orifice central 255 à la périphérie extérieure du plateau. Cette disposition autorise un retrait de chaque plateau 250 sans avoir à retirer la tige verticale 290. Bien entendu dans ce cas, la jupe radialement interne 256 ne couvre pas 360°. Elle est reliée à la jupe radialement externe 254 par deux murets verticaux, de même hauteur que lesdites jupes, rectilignes, et qui encadrent ladite gorge.

De préférence, l'ensemble des moyens composant l'enceinte 100 et l'ensemble 200 sont réalisés en métal, avantageusement en acier inoxydable.

De préférence, le volume interne de l'enceinte est de l'ordre de 500 litres.

L'homme de l'art comprendra que la présente invention permet d'assurer dans un milieu confiné et par conséquent permettant d'éviter toute contamination, l'ensemble des étapes de stérilisation, ensemencement et culture. Le dispositif conforme à la présente invention n'est cependant pas limité à la mise en oeuvre de ces étapes. Il permet également notamment des étapes poste culture, par exemple des étapes de séchage, neutralisation, etc ..., typiquement en contrôlant la température interne de l'enceinte dans une gamme de température de 4 à 120°C.

La présente invention permet également de cultiver simultanément dans l'enceinte, sur des plateaux respectifs différents, des cellules ou micro-organismes différents. A cette fin, pour éviter toute contamination d'une culture par celle d'un autre plateau, l'enceinte est conçue pour contrôler un flux de gaz injecté dans la cuve, notamment un flux d'oxygène, de sorte que ce flux assure de manière optimale la culture, sans flux additionnel superflus susceptible d'assurer l'entraînement ou déplacement de micro-organismes au sein de l'enceinte.

Bien entendu l'ensemble de fluide et gaz extrait de l'enceinte sont de préférence filtrés pour éviter toute contamination de l'environnement.

Par ailleurs, toutes les entrées et sorties de l'enceinte susceptibles de commuter entre deux types de fluide ou gaz sont de préférence équipées de vannes double ou multiple afin de garantir la synchronisation entre les commutations des différents fluides et gaz.

En particulier, l'enceinte peut porter tout équipement additionnel (manomètre, vannes d'entrée ou de sortie, filtres, sondes, etc...) comme schématisé sur la figure 8 à titre non limitatif, pour contrôler le bon fonctionnement du dispositif.

## Revendications

1. Dispositif destiné à la culture de cellules ou micro-organismes comprenant une enceinte (100) constituée d'une cuve (110) et d'un couvercle (150), adaptée pour définir à l'état fermé un volume étanche sous pression contrôlée, et un ensemble (200) conçu pour être placé à l'intérieur de l'enceinte (100) tout en étant susceptible d'être retiré de celle-ci lors de l'ouverture du couvercle (150), l'enceinte (100) comprenant par ailleurs des moyens (113, 160, 130, 114) conçus pour assurer successivement, la stérilisation de son contenu, l'ensemencement d'un milieu de culture placé dans les plateaux (250) et un contrôle de l'atmosphère de l'enceinte (100) propre à permettre la culture de cellules ou micro-organismes, **caractérisé en ce que** l'ensemble comprend en outre un châssis support (210) et une pluralité de plateaux (250) portés par le châssis support (210), et **en ce que** le dispositif comprend des moyens (280) conçus pour assurer l'homogénéisation du milieu de culture et/ou de l'ensemencement de celui-ci, les moyens d'homogénéisation comportant des tiges (282) dont une extrémité libre repose sur une jupe (254) des plateaux (250).

2. Dispositif selon la revendication 1, **caractérisé en ce qu'**il comprend également des moyens (113) conçus pour assurer un traitement après la culture, de préférence un traitement thermique apte à sécher le milieu de culture ainsi que les cellules ou micro-organismes cultivés.

3. Dispositif selon l'une des revendications 1 ou 2, **caractérisé en ce que** l'interface définie entre la cuve (110) et le couvercle (150) de l'enceinte (100) est une interface comprenant un joint annulaire d'étanchéité.

4. Dispositif selon l'une des revendications 1 à 3, **caractérisé en ce que** le châssis support (210) est relié au couvercle (150) de l'enceinte (100), de préférence par des moyens de liaison amovibles.

5. Dispositif selon l'une des revendications 1 à 4, **caractérisé en ce que** l'enceinte (100) est conçue pour contrôler un flux de gaz injecté dans la cuve, notamment un flux d'oxygène, de sorte que ce flux assure de manière optimale la culture, sans flux additionnel susceptible d'entraîner des cellules ou micro-organismes.

6. Dispositif selon l'une des revendications 1 à 5, **caractérisé en ce que** la cuve (110) comporte une double enveloppe (113) définissant une chambre permettant, lorsqu'elle est alimentée en un fluide à température contrôlée, de contrôler de manière homogène la température du volume interne de l'enceinte (100).

7. Dispositif selon l'une des revendications 1 à 6, **caractérisé en ce que** l'enceinte (100) comporte un hublot (120) s'étendant sur toute la hauteur de l'ensemble (200) comportant des plateaux (250).

8. Dispositif selon l'une des revendications 1 à 7, **caractérisé en ce que** l'enceinte (100) comporte des moyens (130) permettant l'introduction d'aiguilles assurant l'ensemencement d'un milieu de culture ou le prélèvement de tout ou partie du contenu des plateaux.

9. Dispositif selon la revendication 8, **caractérisé en ce que** les moyens (130) permettant l'introduction d'aiguilles sont formés de bouchons (132) en matériau élastomère.

10. Dispositif selon la revendication 9, **caractérisé en ce que** les bouchons (132) en matériau élastomère sont montés amovibles.

11. Dispositif selon l'une des revendications 1 à 10, **caractérisé en ce que** l'enceinte (100) comporte un nombre de moyens (130) permettant l'introduction d'aiguilles assurant l'ensemencement d'un milieu de culture, égal au nombre de plateaux (250) porté par le châssis support.

12. Dispositif selon l'une des revendications 1 à 11, **caractérisé en ce que** l'enceinte (100) un orifice de vidange (115) à sa base.

13. Dispositif selon l'une des revendications 1 à 12, **caractérisé en ce que** l'enceinte (100) comporte au moins une entrée (160) permettant d'injecter un fluide ou gaz approprié.

14. Dispositif selon l'une des revendications 1 à 13, **caractérisé en ce que** l'enceinte (100) comporte des sondes dédiées au contrôle de toute les paramètres à l'intérieur de la cuve, et au moins une sonde de température (118), adaptée pour mesurer la température interne de l'enceinte.

15. Dispositif selon l'une des revendications 1 à 14, **caractérisé en ce que** l'enceinte (100) comporte des moyens de mesure de température adaptés pour être placés au sein du milieu de culture portés par les plateaux (250).

16. Dispositif selon l'une des revendications 1 à 15, **caractérisé en ce que** l'enceinte (100) comporte des moyens de mesure de température du type à transmission sans fil.

17. Dispositif selon l'une des revendications 1 à 16, **caractérisé en ce qu'**il comporte des moyens d'éclairage du volume interne de l'enceinte (100).

18. Dispositif selon l'une des revendications 1 à 17, **caractérisé en ce que** le couvercle (150) de l'enceinte (100) porte des moyens d'éclairage et d'observation de l'intérieur de la cuve.

19. Dispositif selon l'une des revendications 1 à 18, **caractérisé en ce qu'**il comporte des moyens d'éclairage au niveau d'un hublot (120).

20. Dispositif selon l'une des revendications 1 à19, **caractérisé en ce que** le châssis support (210) est composé de plusieurs colonnes verticales (220), reliées entre elles par des jeux de traverses ou barreaux horizontaux (222) servant de support aux plateaux (250).

21. Dispositif selon la revendication 1, **caractérisé en ce que** les moyens (280) conçus pour assurer l'homogénéisation du milieu de culture et/ou de l'ensemencement de celui-ci comprennent des palles ou râteaux (280) susceptibles d'être entraînés en rotation.

22. Dispositif selon l'une des revendications 1 à 21, **caractérisé en ce** les plateaux (250) possèdent en leur centre un orifice (255) surmonté d'une jupe cylindrique (256) permettant le passage d'une tige (290) conçue pour l'entraînement de moyens d'homogénéisation.

23. Dispositif selon l'une des revendications 1 à 21, **caractérisé en ce** chaque plateau (250) possède une gorge radiale.

## Claims

1. Device intended for the culture of cells or micro-organisms comprising a chamber (100) composed of a tank (110) and a lid (150), adapted so as to define in the closed state a sealed volume under a controlled pressure, and an assembly (200) designed to be placed inside the chamber (100) while being capable of being removed therefrom when the lid (150) is opened, the chamber (100) also comprising means (113, 160, 130, 114) for successively sterilising the contents thereof, seeding a culture medium placed in the plates (250) and controlling the atmosphere of the chamber (100) so as to allow the culture of cells or micro-organisms, **characterised in that** the set further comprises a support frame (210) and a plurality of plates (250) carried by the support frame (210), and **in that** the means designed to homogenise comprising rods (282) of which one free end rests on a skirt (254) of the plates (750).

2. Device according to claim 1, **characterised in that** it also comprises means (113) designed to carry out a treatment after the culture, preferably a heat treatment capable of drying the culture medium and also the cultured cells or micro-organisms.

3. Device according to one of claims 1 or 2, **characterised in that** the interface defined between the tank (110) and the lid (150) of the chamber (100) is an interface comprising an annular seal.

4. Device according to one of claims 1 to 3, **characterised in that** the support frame (210) is connected to the lid (150) of the chamber (100), preferably by removable connecting means.

5. Device according to one of claims 1 to 4, **characterised in that** the chamber (100) is designed to control a stream of gas injected into the tank, in particular a stream of oxygen, so that this stream performs the culture in an optimal manner, without any additional stream likely to entrain cells or micro-organisms.

6. Device according to one of claims 1 to 5, **characterised in that** the tank (110) comprises a double casing (113) defining an enclosure which, when it is supplied with a fluid at a controlled temperature, makes it possible to control in a homogeneous manner the temperature of the internal volume of the chamber (100).

7. Device according to one of claims 1 to 6, **characterised in that** the chamber (100) comprises a window (120) extending over the entire height of the assembly (200) comprising plates (250).

8. Device according to one of claims 1 to 7, **characterised in that** the chamber (100) comprises means (130) allowing the introduction of needles for seeding a culture medium or removing all or part of the contents of the plates.

9. Device according to claim 8, **characterised in that** the means (130) allowing the introduction of needles are formed by stoppers (132) made from elastomeric material.

10. Device according to claim 9, **characterised in that** the stoppers (132) made from elastomeric material are mounted in a removable manner.

11. Device according to one of claims 1 to 10, **characterised in that** the chamber (100) comprises a number of means (130) allowing the introduction of needles for seeding a culture medium, equal to the number of plates (250) carried by the support frame.

12. Device according to one of claims 1 to 11, **characterised in that** the chamber (100) has a drainage orifice (115) at its base.

13. Device according to one of claims 1 to 12, **characterised in that** the chamber (100) comprises at least one inlet (160) making it possible to inject a suitable fluid or gas.

14. Device according to one of claims 1 to 13, **characterised in that** the chamber (100) comprises probes dedicated to controlling all the parameters inside the tank, and at least one temperature probe (118) for measuring the internal temperature of the chamber.

15. Device according to one of claims 1 to 14, **characterised in that** the chamber (100) comprises temperature measurement means suitable for being placed within the culture medium carried by the plates (250).

16. Device according to one of claims 1 to 15, **characterised in that** the chamber (100) comprises temperature measurement means of the wireless transmission type.

17. Device according to one of claims 1 to 16, **characterised in that** it comprises means for illuminating the internal volume of the chamber (100).

18. Device according to one of claims 1 to 17, **characterised in that** the lid (150) of the chamber (100) carries means for illuminating and observing the interior of the tank.

19. Device according to one of claims 1 to 18, **characterised in that** it comprises illumination means at a window (120).

20. Device according to one of claims 1 to 19, **characterised in that** the support frame (210) is composed of a plurality of vertical columns (220), connected to one another by sets of crossbars or horizontal bars (222) serving to support the plates (250).
The device comprises means (280) designed to homogenise the culture medium and/or the seeding thereof, the means designed to homogenise comprising rods (282) of which one free end rests on a skirt (254) of the plates (750).

21. Device according to claim 1, **characterised in that** the means (280) designed to homogenise the culture medium and/or the seeding thereof comprise blades or spreaders (280) capable of being driven in rotation.

22. Device according to one of claims 1 to 21, **characterised in that** the plates (250) have in their centre an orifice (255) surmounted by a cylindrical skirt (256) allowing the passage of a rod (290) designed to drive the homogenisation means.

23. Device according to one of claims 1 to 21, **characterised in that** each plate (250) has a radial groove.

## Patentansprüche

1. Vorrichtung zur Kultivierung von Zellen oder Mikroorganismen, umfassend eine Einfassung (100), die aus einem Behälter (110) und einem Deckel (150) besteht und dazu geeignet ist, um im geschlossenen Zustand ein dichtes Volumen unter geregeltem Druck zu bilden, und eine Anordnung (200), die dazu ausgelegt ist, um im Innern der Einfassung (100) angeordnet zu werden, und dabei bei Öffnung des Deckels (150) aus dieser entnehmbar ist, wobei die Einfassung (100) ferner Mittel (113, 160, 130, 114) umfasst, die dazu ausgelegt sind, um nacheinander die Sterilisierung ihres Inhalts, die Einimpfung eines Nährbodens, der auf den Böden (250) angeordnet ist, und eine Regelung der Atmosphäre der Einfassung (100) derart, dass sie die Kultivierung von Zellen oder Mikroorganismen ermöglicht, sicherzustellen, **dadurch gekennzeichnet, dass** die Anordnung ferner ein Traggestell (210) und eine Vielzahl von Böden (250) umfasst, die von dem Traggestell (210) getragen werden, und dass die Vorrichtung Mittel (280) umfasst, die dazu ausgelegt sind, um die Homogenisierung des Nährbodens und/oder der Einimpfung desselben sicherzustellen, wobei die Homogenisierungsmittel Stäbe (282) umfassen, von denen ein freies Ende auf einem Mantel (254) der Böden (250) aufliegt.

2. Vorrichtung nach Anspruch 1, **dadurch gekennzeichnet, dass** sie auch Mittel (113) umfasst, die dazu ausgelegt sind, um eine Behandlung nach der Kultivierung sicherzustellen, bevorzugt eine Wärmebehandlung, die dazu geeignet ist, den Nährboden sowie die kultivierten Zellen oder Mikroorganismen zu trocknen.

3. Vorrichtung nach einem der Ansprüche 1 oder 2, **dadurch gekennzeichnet, dass** die Schnittstelle, die zwischen dem Behälter (110) und dem Deckel (150) der Einfassung (100) definiert ist, eine Schnittstelle ist, die eine ringförmige Dichtung umfasst.

4. Vorrichtung nach einem der Ansprüche 1 bis 3, **dadurch gekennzeichnet, dass** das Traggestell (210) mit dem Deckel (150) der Einfassung (100) bevorzugt über abnehmbare Verbindungsmittel verbunden ist.

5. Vorrichtung nach einem der Ansprüche 1 bis 4, **dadurch gekennzeichnet, dass** die Einfassung (100) dazu ausgelegt ist, um einen Gasstrom zu regeln, der in den Behälter eingeblasen wird, insbesondere einen Sauerstoffstrom, so dass dieser Strom die Kultur auf optimale Art und Weise sicherstellt, ohne einen zusätzlichen Strom, der Zellen oder Mikroorganismen mitnehmen könnte.

6. Vorrichtung nach einem der Ansprüche 1 bis 5, **dadurch gekennzeichnet, dass** der Behälter (110) eine doppelte Verkleidung (113) umfasst, die eine Kammer definiert, die es ermöglicht, wenn sie mit einem Fluid auf geregelter Temperatur versorgt wird, die Temperatur des Innenvolumens der Einfassung (100) einheitlich zu regeln.

7. Vorrichtung nach einem der Ansprüche 1 bis 6, **dadurch gekennzeichnet, dass** die Einfassung (100) ein Fenster (120) umfasst, das sich über die gesamte Höhe der die Böden (250) umfassenden Anordnung (200) hinweg erstreckt.

8. Vorrichtung nach einem der Ansprüche 1 bis 7, **dadurch gekennzeichnet, dass** die Einfassung (100) Mittel (130) umfasst, welche die Einführung von Nadeln ermöglichen, welche die Einimpfung eines Nährbodens oder die Entnahme des gesamten Inhalts der Böden, oder eines Teils davon, sicherstellen.

9. Vorrichtung nach Anspruch 8, **dadurch gekennzeichnet, dass** die Mittel (130), welche die Einführung von Nadeln ermöglichen, aus Stopfen (132) aus einem elastomeren Material gebildet sind.

10. Vorrichtung nach Anspruch 9, **dadurch gekennzeichnet, dass** die Stopfen (132) aus einem elastomeren Material abnehmbar angebracht sind.

11. Vorrichtung nach einem der Ansprüche 1 bis 10, **dadurch gekennzeichnet, dass** die Einfassung (100) eine Anzahl von Mitteln (130) umfasst, welche die Einführung von Nadeln ermöglichen, welche die Einimpfung eines Nährbodens sicherstellen, die gleich der Anzahl von Böden (250) ist, die von dem Traggestell getragen werden.

12. Vorrichtung nach einem der Ansprüche 1 bis 11, **dadurch gekennzeichnet, dass** die Einfassung (100) an ihrer Basis eine Ablauföffnung (115) umfasst.

13. Vorrichtung nach einem der Ansprüche 1 bis 12, **dadurch gekennzeichnet, dass** die Einfassung (100) mindestens einen Eingang (160) umfasst, der es ermöglicht, ein geeignetes Fluid oder Gas einzubringen.

14. Vorrichtung nach einem der Ansprüche 1 bis 13, **dadurch gekennzeichnet, dass** die Einfassung (100) Sonden umfasst, die zur Regelung aller Parameter im Innern des Behälters bestimmt sind, und mindestens eine Temperatursonde (118), die dazu geeignet ist, um die Innentemperatur der Einfassung zu messen.

15. Vorrichtung nach einem der Ansprüche 1 bis 14, **dadurch gekennzeichnet, dass** die Einfassung (100) Temperaturmessmittel umfasst, die dazu geeignet sind, um in dem Nährboden angeordnet zu werden, der von den Böden (250) getragen wird.

16. Vorrichtung nach einem der Ansprüche 1 bis 15, **dadurch gekennzeichnet, dass** die Einfassung (100) Temperaturmessmittel von der Art mit drahtloser Übertragung umfasst.

17. Vorrichtung nach einem der Ansprüche 1 bis 16, **dadurch gekennzeichnet, dass** sie Mittel zum Beleuchten des Innenvolumens der Einfassung (100) umfasst.

18. Vorrichtung nach einem der Ansprüche 1 bis 17, **dadurch gekennzeichnet, dass** der Deckel (150) der Einfassung (100) Mittel zum Beleuchten und Beobachten des Innern des Behälters trägt.

19. Vorrichtung nach einem der Ansprüche 1 bis 18, **dadurch gekennzeichnet, dass** sie Mittel zur Beleuchtung an einem Fenster (120) umfasst.

20. Vorrichtung nach einem der Ansprüche 1 bis 19, **dadurch gekennzeichnet, dass** das Traggestell (210) aus mehreren senkrechten Kolonnen (220) besteht, die untereinander durch Gruppen von Querverbindungen oder waagerechten Stangen (222) verbunden sind, die zur Abstützung der Böden (250) dienen.

21. Vorrichtung nach Anspruch 1, **dadurch gekennzeichnet, dass** die Mittel (280), die ausgelegt sind, um die Homogenisierung des Nährbodens und/oder die Einimpfung desselben sicherzustellen, Schaufeln oder Rechen (280) umfassen, die drehbar mitgenommen werden können.

22. Vorrichtung nach einem der Ansprüche 1 bis 21, **dadurch gekennzeichnet, dass** die Böden (250) in ihrer Mitte eine Öffnung (255) besitzen, über der sich ein zylindrischer Mantel (256) befindet, der den Durchgang eines Stabs (290) ermöglicht, der dazu ausgelegt ist, um die Homogenisierungsmittel anzutreiben.

23. Vorrichtung nach einem der Ansprüche 1 bis 21, **dadurch gekennzeichnet, dass** jeder Boden (250) eine radiale Nut besitzt.
